# EUROPEAN PATENT SPECIFICATION

(11) **EP 2 227 273 B1**
(45) Date of publication and mention of the grant of the patent: **13.01.2016**
(21) Application number: 08851373.4
(22) Date of filing: 21.11.2008
(51) Int. Cl.: A61M 3/02

(54) **CANNULA FOR DISPENSING FLUID PRODUCTS, PARTICULARLY FOR RECTAL APPLICATIONS**
KANÜLE ZUR ABGABE VON FLÜSSIGEN PRODUKTEN, INSBESONDERE FÜR REKTALE ANWENDUNGEN
CANULE DE DISTRIBUTION DE PRODUITS FLUIDES, EN PARTICULIER POUR APPLICATIONS RECTALES

(30) Priority: 23.11.2007 IT MO20070357; 23.11.2007 IT MO20070358
(43) Date of publication of application: 15.09.2010
(73) Proprietor: Lameplast S.P.A., Frazione Rovereto Sul Secchia (IT)
(72) Inventor: FONTANA, Antonio, 41012 Carpi (MO) (IT)
(74) Representative: Brunacci, Marco
(86) International application number: PCT/IB2008/003172
(87) International publication number: WO 2009/066163

(56) References cited:
- EP-A- 0 139 855
- EP-B- 0 263 976
- WO-A-98/34671
- WO-A-2006/134464
- US-A- 5 219 448

## Description

### Technical Field

This invention relates to a cannula for dispensing fluid products, particularly for rectal applications.

### Background Art

Various hygienic-sanitary devices exist for the dispensing of medicinal fluid products, specifically designed for rectal applications.

One of these devices consists of traditional enemas which, in point of fact, consist of a deformable pear-shaped flexible-rubber bag that terminates with a dispenser pipe.

The enemas are filled with the medicinal product before use; following the squeezing of the flexible-rubber bag, therefore, the product to be administered is pushed inside the dispenser pipe from which it exits through an opening in the extremity.

Another device of known type is composed of plastic bottles shaped like a bellows, which also have a dispenser pipe but which, unlike the enemas, do not have a flexible-rubber bag, but a container that folds on itself like a bellows. These bottles work in the same way as the enemas, because in the case of the bottles as well, the product is dispensed through the pipe by squeezing the bellows container.

Both the enemas and the bottles, however, have various drawbacks, such as, for example, the fact that they are affected by rather high overall dimensions and inappropriate limitations as regards use.

In this respect, the fact is emphasised that to apply products in fluid state in the form of paste or cream, these devices of known type are particularly inconvenient and awkward to use and, in some cases, are totally inefficient. Furthermore, it must not be forgotten that the dimensions and the shape of these devices do not always allow dispensing the totality of the products contained in them, which, on the contrary, remains trapped, at least in part, in the flexible-rubber bag or in the bellows container, thus representing a pointless waste.

Other devices are also known for the application of medical fluids made up of a tube, containing the product to be dispensed, and an internally hollow dispensing rod, which is initially empty and separated from the tube.

The tube has an exit opening for the fluid product which has a threaded spout onto which the dispensing rod is screwed before use.

The dispensing rod, in turn, has a series of openings obtained on its side surface. During the application phase therefore, the squeezing of the tube pushes the fluid product along the dispensing rod until it reaches its side openings, through which the fluid product is distributed directly in contact with the walls of the body cavity to be medicated.

This particular type of device does however also have several drawbacks related, in particular, to the considerable difficulties associated with use and to the impossibility, as in the case of the enemas and bottles already illustrated, to dispense all the medical product contained in the tube.

Inside the dispensing rod, in fact, at least partially trapped, remains a certain quantity of fluid to be dispensed; such quantity cannot be administered and is pointlessly wasted.

Another sanitary instrument is known from WO 2006/134464, which discloses a syringe-like device substantially similar to the previous device, with the exception that the squeezable tube containing the fluid product is substituted by a rigid hollow and cylindrical body in which a piston can slide.

The rigid body is suitable to support the dispensing rod, through which the fluid product can be dispensed under the pressure of the piston.

However, the rigid body according to WO 2006/134464 has the same drawbacks of the squeezable tube because the piston can move only inside the rigid body and not inside the dispenser rod; consequently, a part of the fluid product remains at least partially trapped inside the dispensing rod and is wasted.

Also known are cannulas which, generally, are sold in packs together with tubes or bottles containing the product to be applied.

The known cannulas are composed of a cylinder suitable for containing the product and inside which slides a piston integral with the extremity of a push rod.

The piston is usually composed of a flat-shaped plate fitted sliding along the inner side walls of the cannula.

The cylinder has an open extremity that can be coupled with the dispenser mouth of the tube, to introduce into the cylinder itself the quantity of the product to be applied, and through which the introduced product is dispensed.

The opposite extremity of the cylinder is closed by a bottom which is provided with a hole, in which the push rod is fitted sliding, and which acts as a stop element for the piston to prevent this slipping out.

The product introduced into the cannula is dispensed by operating the push rod in the direction of movement of the piston towards the open extremity of the cylinder.

Another type of cannula is composed of a cylinder, which has its opposite extremities open and inside which is slidingly located a plate-shaped piston, and of a push rod separated from the piston and which can be coupled to this in a removable way.

At the opposite extremities of the cylinder, undercuts or stop shoulders are obtained suitable for stopping piston sliding and preventing the piston from coming out as a result of the action of the push rod.

These latter cannulas can be sold in packs containing a single push rod, a plurality of empty disposable cylinders to be used for the different applications and one or more tubes of product.

In this case one of the two extremities of the cylinders can be coupled with the dispenser mouth of the tube in order to introduce the product inside the cylinders themselves, while the opposite extremity acts as a passage for the push rod.

Alternatively, these cannulas can be sold in packs containing a single push rod and a plurality of cylinders already filled with the product to be applied and having two opposite extremities with closing caps that are removed at the time of use.

The cannulas of known type are however also liable to further targeted upgrading, in particular, to make them more practical and easier to use.

In this respect, it should be noted that the traditional cannulas, having just one dispenser opening placed axially at the free extremity of the cannula, make it possible to allow the product to be dispensed to flow in a longitudinal direction only.

This considerably restricts use considering that some medical products need to be distributed and/or spread over larger surface extensions.

Furthermore, traditional cannulas are often not easy to handle and practical to use.

It should not be forgotten moreover that traditional cannulas are sometimes very complicated to make and, therefore, involve rather high production costs that negatively affect the retail price to the public.

Other kinds of applicators are known from patent documents US 5.219,448, WO 98/34671, EP 0 263 976, WO 2006/134464 and EP 0 139 855.

### Object of the Invention

The main aim of the present invention is to provide a cannula for dispensing fluid products, particularly for rectal applications, that allows achieving the above-mentioned standards of upgrading, that has compact overall dimensions and is very easy to handle, which can be simply and promptly used by users, and which is quick to manufacture, make up and package.

Another object of the present invention is to provide a cannula for dispensing fluid products, particularly for rectal applications, that allows overcoming the mentioned drawbacks of the state of the art as part of a simple and rational solution, that is easy and effective to use and of low cost.

The above objects are achieved by the present cannula for dispensing fluid products, particularly for rectal applications, according to the contents of claim 1.

### Short Description of the Drawings

Other characteristics and advantages of the present invention will become clearer from the description below of a number of illustrative embodiments of a cannula for dispensing fluid products, particularly for rectal applications, including but not limited to those shown in the attached drawings in which:
the figure 1 is an exploded view of a cannula according to the invention;
the figure 2 is an axonometric view of the cannula according to figure 1 in the initial packaging configuration;
the figure 3 is a schematic and partial section view of the cannula according to figures 1 to 2 in the initial packaging configuration;
the figure 4 is an axonometric, schematic and partial view of the cannula according to the figures 1 to 3 in the final complete dispensing configuration;
the figure 5 is a section, schematic and partial view of the cannula according to the figures 1 to 4 in the final complete dispensing configuration.
the figure 6 is a side view of a second cannula according to the invention;
the figure 7 is a side view of a third cannula according to the invention;
the figure 8a is a side view of a fourth cannula according to the invention;
the figure 8b is a section through the cannula of figure 8a after use
the figure 9a is a side view of a fifth cannula according to the invention; and the figure 9b is a section through the cannula of figure 9a .

### Embodiments of the Invention

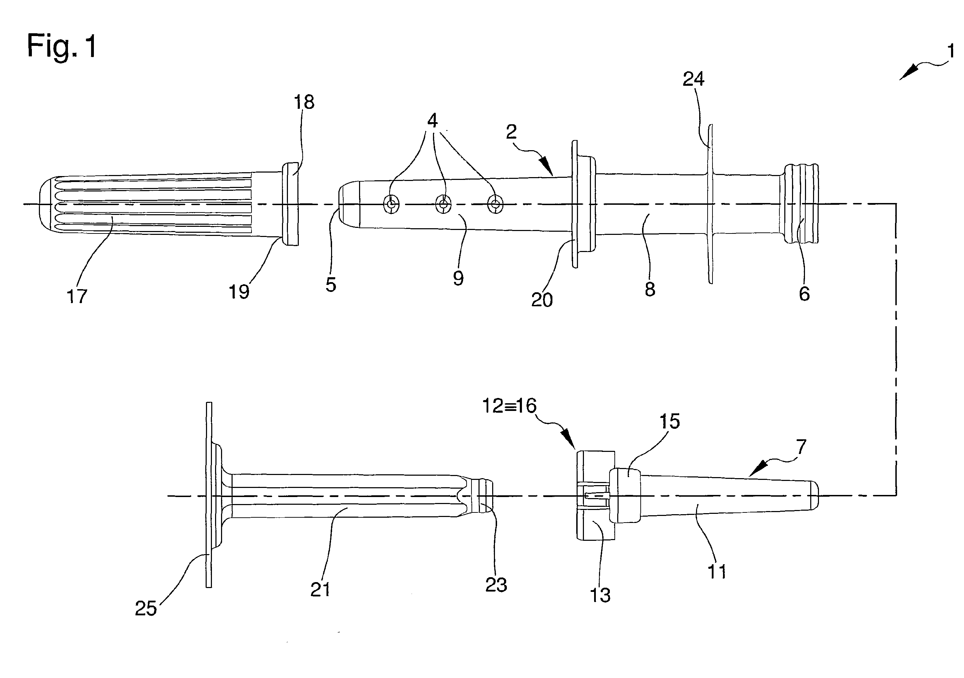

With particular reference to figures 1 to 5, the reference numeral 1 indicates a cannula for dispensing fluid products, particularly for rectal applications.

The cannula 1 comprises a round-section tubular body 2 suitable for containing a product 3 to be dispensed which is in fluid state and, for example, is of the type of liquids, creams, pastes or the like.

The tubular body 2 is at least in part pre-filled, i.e., it is more or less completely filled with the product 3 during the packaging phase before the cannula 1 is distributed on the market.

The tubular body 2 has a plurality of dispensing mouths 4 for dispensing the product 3 provided on its side surface and has a closed axial extremity 5 and an open axial extremity 6.

Through the open axial extremity 6 in the tubular body 2 a piston 7 is fitted, axially slidable, which piston is movable between an initial packaging configuration and a final complete dispensing configuration.

In detail, the tubular body 2 consists of a first portion 8, cylindrical in shape, that starts with the open axial extremity 6 and along which the piston 7 slides, and a second portion 9 that protrudes from the first portion 8, ends in the closed axial extremity 5, and along which are provided the dispensing mouths or lateral outlets 4.

The second portion 9 has a substantially truncated-cone shape, in the sense that, near the closed axial extremity 5, it has a slightly narrower cross section than the cross section near the first portion 8.

In the same way, the piston 7 consists of a first section 10, cylindrical in shape, that slides inside the first portion 8 of the tubular body 2, and of a second section 11 with a truncated-cone shape, which protrudes from the first section 10 and is substantially elongated in the longitudinal direction of the tubular body 2.

The piston 7 and the second portion 9 of tubular body 8 are illustrative of preferred embodiments of the present invention in which the piston is so configured and dimensioned that, in the final dispensing configuration, the outer surface of the piston is substantially contiguous with the inner surface of the tubular body in the region of the tubular body in which at least one dispensing mouth is located. In particular, where the piston outer surface and the inner surface of the tubular body are each slightly convergent, that allows material to continue to exit from all outlets, thereby reducing the tendency for the fluid product to be predominantly expressed from the outlets nearest the closed end. In the final complete dispensing configuration, the second section 11 can be placed substantially in line with the inner surface of the second portion 9 of the tubular body 2.

The second section 11, in point of fact, is designed to push the product 3 through the dispensing mouths 4, moving from the initial packaging configuration to the final complete dispensing configuration.

During forward movement, the truncated-cone shaped surface of the second section 11 permits giving the product 3 both an axial push and a sideways push towards the dispensing mouths 4, allowing the substantial totality of the product 3 to come out of the tubular body 2.

At the same time, the shape of the second section 11 allows obtaining the complete occlusion of the dispensing mouths 4 once the final complete dispensing configuration has been achieved, so as to prevent any return flows of the product 3 inside the second portion 9.

Alternative forms cannot however be ruled out in which the shape of the second portion 9 and of the second section 11, instead of having a truncated-cone shape, is substantially cylindrical. Advantageously, in the initial packaging configuration, the first section 10 of the piston 7 is fitted in the tubular body 2 and, in point of fact, represents its closing bottom which prevents the product 3 from coming out through the open axial extremity 6.

At the open axial extremity 6, temporary retaining means 12 are provided for temporarily retaining the piston 7 in the initial packaging configuration which are interposed in between the first portion 8 of the tubular body 2 and the first section 10 of the piston 7.

The temporary retaining means 12, for example, comprise a strip 13 fitted around the open axial extremity 6 and associated with the piston 7 along predetermined fracture areas 14.

The strip 13 is of the tear-off type and has a grip and tear-off flap 15 suitable for breaking the strip 13 to remove it from the tubular body 2.

During the removal of the strip 13, the predetermined fracture areas 14 are suitable for breaking, leaving the piston 7 free to slide inside the tubular body 2. Expediently, first anti-tampering means 16 are also provided, suitable for sealing the initial packaging configuration.

The first anti-tampering means 16 are interposed between the tubular body 2 and the piston 7 and, in the particular embodiment of the invention shown in the illustrations, coincide with the temporary retaining means 12.

The presence of the strip 13 attached to the tubular body 2 does in fact show the integrity of the cannula 1, proving that this has not been tampered with after fabrication and that the product 3 has not been damaged in any way inside. Alternative embodiments cannot however be ruled out in which the first anti-tampering means 16 are different and distinct from the temporary retaining means 12.

Advantageously, the dispensing mouths 4 are round shaped and split up into a first series distributed longitudinally on the tubular body 2 and into a second series distributed longitudinally diametrically opposite the first series.

Examples are however possible in which there is only one dispensing mouth 4 which, for example, is elongated in the longitudinal direction of the tubular body 2 or has a helical shape that wraps around the second portion 9 like a spiral.

The shape, the dimensions and the position of the dispensing mouths 4 along the tubular body 2 can be differentiated according to the type of product 3 used and to the pharmaceutical treatment to be applied.

This way, in fact, a distribution of the product 3 can be obtained on a more or less ample surface and in a more or less uniform and/or localised way.

To temporarily close the dispensing mouths 4 a cap 17 is provided that can be fitted to measure around the second portion 9 of the tubular body 2.

In between the tubular body 2 and the cap 17 are placed second anti-tampering means 18, suitable for sealing the fastening of the dispensing mouths 4 and underlining the separation of the cap 17 from the tubular body 2.

The second anti-tampering means 18 consist, for example, in an annular element that is associated with the edge of the cap 17 along a tearing line 19 and which can be fixed around the tubular body 2.

For this purpose around the tubular body 2 is rigidly associated a plate 20 that has a groove in which the annular element 18 can be interlocked.

Usefully, the cannula 1 comprises a push rod 21 separated from the piston 7, associable with it and fittable in the tubular body 2 through the open axial extremity 6 to push the piston 7 along the tubular body 2 and dispense the product 3.

Between the piston 7 and the push rod 21 are placed temporary mutual fastening means 22, 23 which, in the embodiment of the invention shown in the figures 1 to 5, consist of a shaped cavity 22 provided inside the first section 10 of the piston 7 and of a shaped protrusion 23 provided at an extremity of the push rod 21 and which can be interlocked into the shaped cavity 22.

During use, the push rod 21 does not come directly into contact with the product 3 but fastens and unfastens onto/from the piston 7 by means of the shaped cavity 22 and the shaped protrusion 23; this way the push rod 21 can be used again with different cannulas 1.

The cannulas 1, therefore, can be distributed on the market both in packs comprising a plurality of push rods 21, one for each tubular body 2 containing the product 3, and in packs containing just one push rod 21 intended for use with a plurality of tubular bodies 2 for operating the corresponding pistons 7. Advantageously, grip means 24, 25 are provided for gripping the tubular body 2 and the push rod 21 that enable the user to use the cannula 1.

The grip means 24, 25, for example, are composed of a first round-shaped pad 24, which extends from the second portion 9 of the tubular body 2 crossways to this, and of a second round-shaped pad 25, which extends crossways from the end extremity of the push rod 21 opposite the shaped protrusion 23.

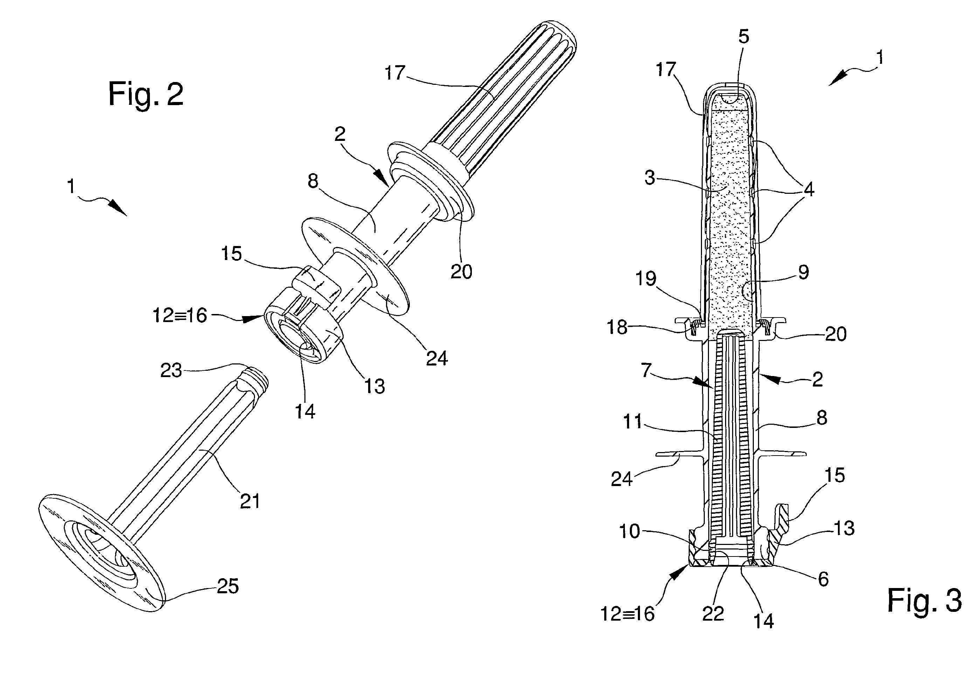

The cannula 1 is sold in the initial packaging configuration (figures 2 and 3) and is pre-filled with the product 3; in this configuration, the push rod 21 is separated from the piston 7, the strip 13 is fitted on the tubular body 2 and is associated with the piston 7, while the cap 17 is fitted on the second portion 9 and is temporarily fastened, by means of the annular part 18, to the plate 20.

To use the cannula 1 the grip and tear-off flap 15 must be torn off, the strip 13 removed, the push rod 21 engaged in the first section 10 of the piston 7, the tearing line 19 broken and the cap 17 removed from the tubular body 2.

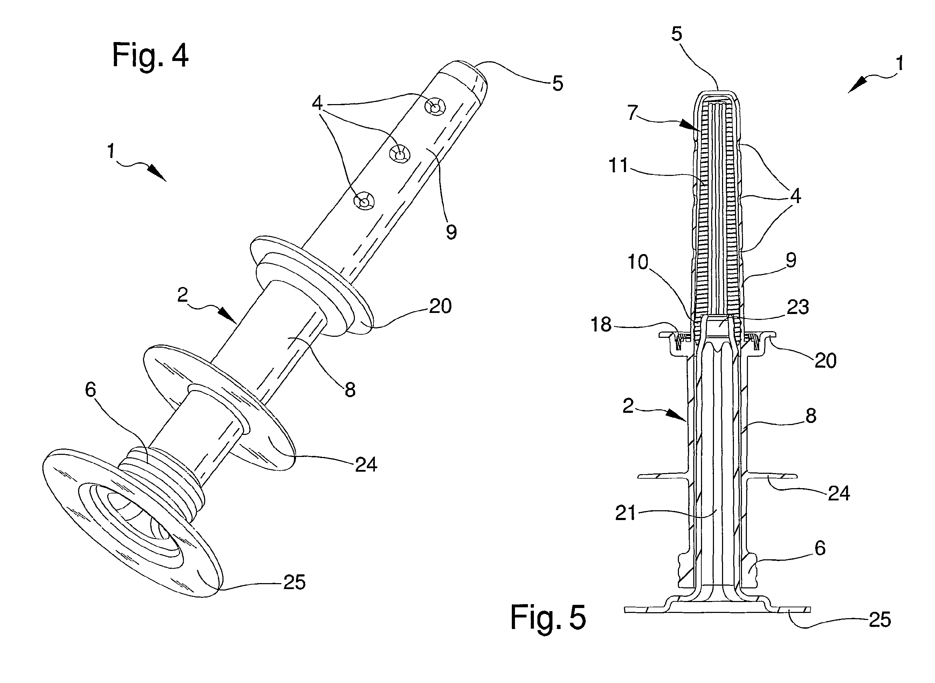

At this point, the cannula 1 is ready for application, which can be done by the simple manual action of the user, by levering on the first and on the second pad 24 and 25, to move the piston 7 as far as the complete dispensing configuration (figures 4 and 5) and expel the product 3 through the dispensing mouths 4.

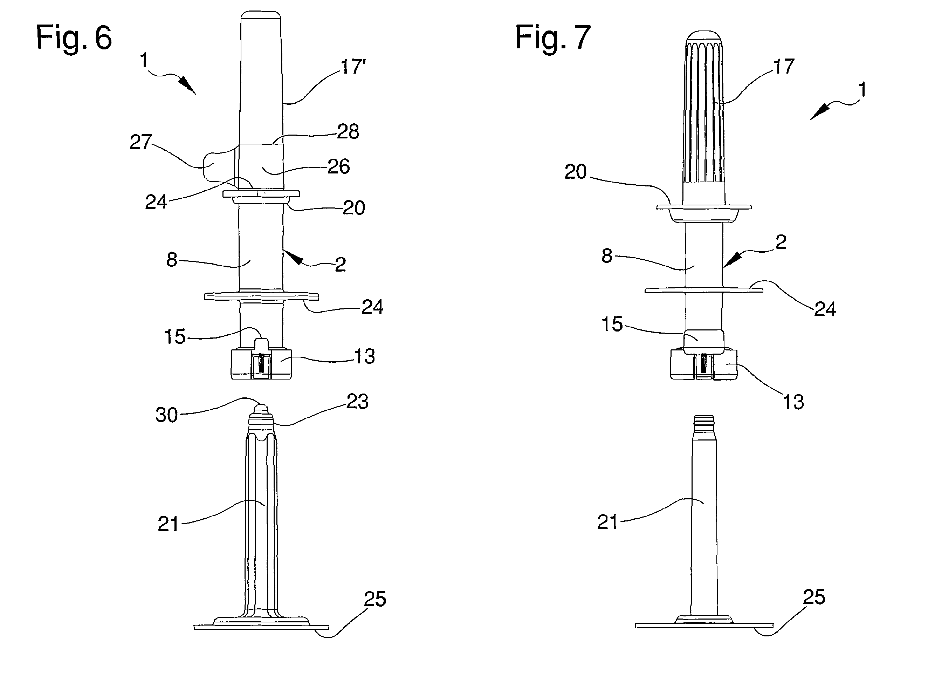

A second Example is shown in figure 6. The cannula of figure 6 is generally of similar construction to that of figures 1 to 5, and the same reference numerals are used in figure 6 to refer to features indicated by those reference numerals in figures 1 to 5. The device of figure 6 has, as tamper-evident means to indicate previous whole or partial cap removal, a tear-around strip 26, with gripping tab 27. The tamper-evident strip 26 is joined to the cap by means of a line of weakening 28, and to the plate 20 via an annular region of weakening 29. The cap 17' differs from the cap 17 shown in figure 1 in having a smooth surface. In the embodiment of figure 6, the push rod 21 is provided at its distal end with a protrusion 30, for example in the form of a rubber grommet, to assist engagement of the push rod 21 with piston 7.

A third Example is shown in figure 7, in which the same reference numerals are used for parts that are shown in figures 1 to 5. The device of figure 7 is identical to that of figures 1 to 5 except that the surface of the push rod is smooth, rather than having elongate ridges and troughs.

The embodiments of Figures 1 to 5, 6 and 7 are illustrative of certain preferred devices according to the invention in which the push rod and piston are reversibly attachable to one another.

Devices arranged to allow reversible attachment of the piston to the push rod have a number of advantages. As already mentioned, it allows the push rod to be re-usable, which provides efficiency and economy. Additionally, it allows the device to be supplied in a pack of length shorter than that required for the assembled configuration, making it, for example, easier to store discreetly in a user's pocket or handbag. Furthermore, the risk of inadvertent actuation of the piston before use is low, and the devices may be easy to fill.

In this case, the cannulas 1 are distributed on the market in packages comprising a piston 7/push rod 21 for each tubular body 2, foreseeing an operation substantially the same as that of the previously described and illustrated cannulas 1.

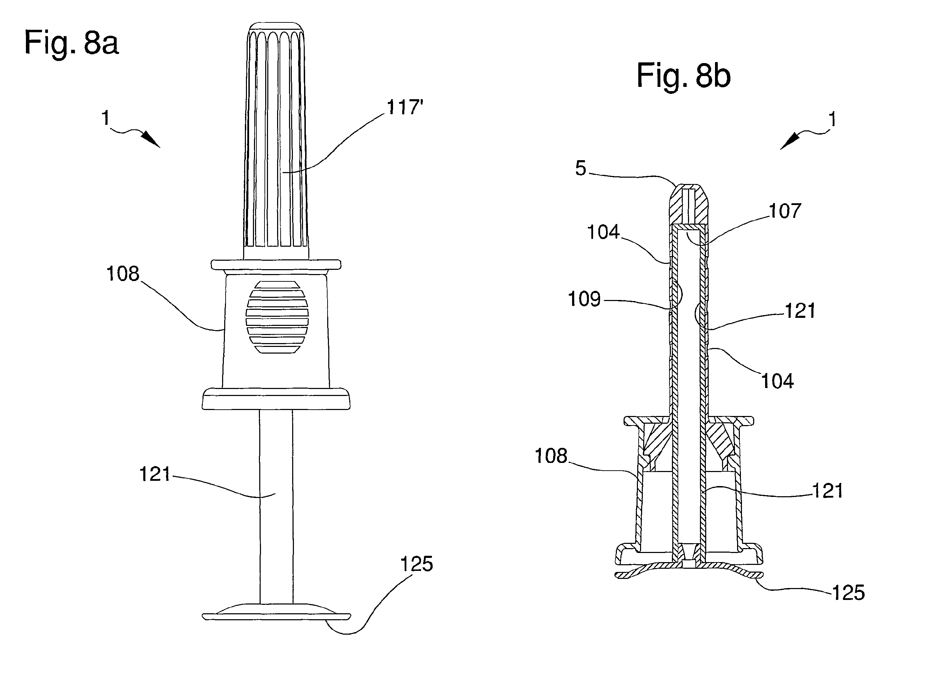

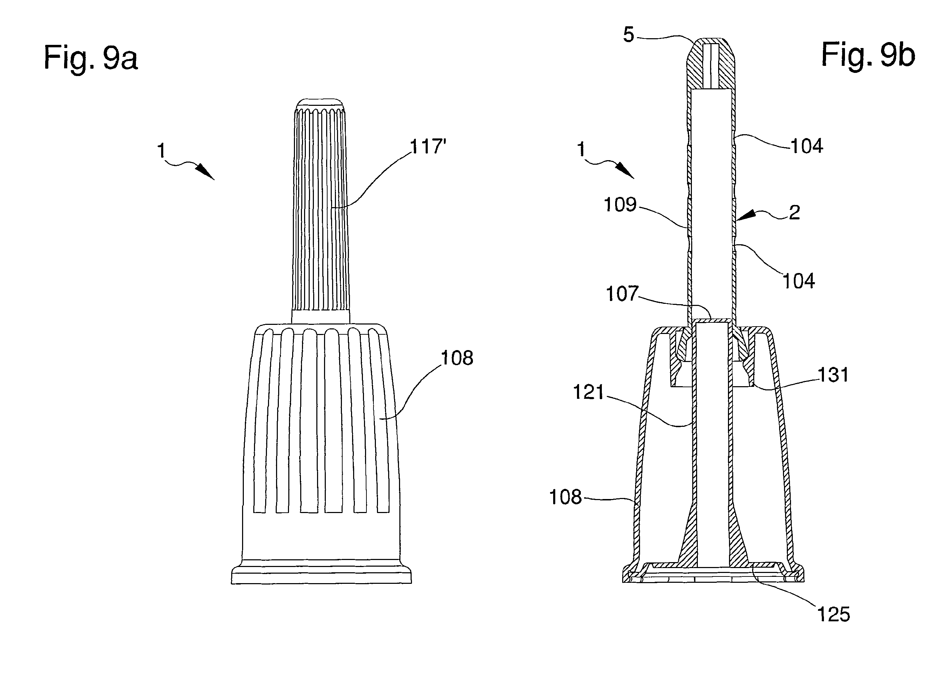

Examples in figures 8a and 8b and in figures 9a and 9b are illustrative of devices in which the push rod and piston are integrally formed. In these figures in the configuration before use (fig. 8a), the device has a cap 117' enclosing second portion 109, along which outlets 104 are provided. The first portion 108 is of enlarged cross-section relative to second portion 109. The piston 107 is integrally formed with a tubular push rod 121, which has at the opposite end to piston 107 a flange 125. The piston can be actuated by depressing the flange 125 into the first portion 108.

Referring to figures 9a and 9b, the one-piece push rod and piston is housed almost wholly within the first portion 108, and on actuation of the piston the flange 125 is pushed into the first portion 108, coming rest on seat 131. Appropriate tamper-evident means may be provided between flange 125 and the adjacent part of the wall of first portion 108

The devices of the invention are preferably of dimensions suitable for use in rectal applications. For example, the devices may advantageously be arranged to deliver from 1 to 25ml, for example 2 to 20ml, especially from 5 to 20ml of fluid product for rectal application.

An example also provides, a cannula for dispensing fluid products, particularly for rectal applications, comprising at least one tubular body for containing a fluid product to be dispensed which has at least a product dispensing mouth and at least an open axial extremity, at least one cap fittable substantially to measure around said tubular body to temporarily close said dispensing mouth, at least one piston fitted axially sliding in said tubular body between an initial packaging configuration and a final dispensing configuration, at least one push rod associable with said piston and fittable in said tubular body through said open axial extremity, characterized in that said dispensing mouth is provided on the side surface of said tubular body.

In one optional form of cannula or device according to the invention the cannula or device does not comprise a substantially tubular liner which is associated with said tubular body.

In another optional form the cannula or device does not comprise a substantially tubular liner which is associable with said tubular body around said push piston, and temporary withholding means for withholding said push piston in said initial packaging configuration which are placed in between said liner and said push piston.

In yet another optional form the cannula or device is not one comprising a tubular body for containing a fluid product to be dispensed which has at least a product dispensing mouth and at least an open axial extremity, at least a cap fittable substantially snugly around said tubular body for temporarily closing said dispensing mouth, at least a push piston for pushing said product which is at least partially fittable in said tubular body through said open axial extremity and moving between an initial packaging configuration and a final dispensing configuration, at least a substantially tubular liner which is associable with said tubular body around said push piston, and temporary withholding means for withholding said push piston in said initial packaging configuration which are placed in between said liner and said push piston, said dispensing mouth being obtained on the side surface of said tubular body.

In a further optional form the cannula or device have a push rod and a piston that are not monolithic.

It has in practice been demonstrated how the described invention achieves the proposed objects.

In this respect, the fact is underlined that the particular solution of providing dispensing mouths arranged on the side surface of the cannula permits the more practical and successful distribution of the product to be dispensed by means of a very simple, compact and inexpensive cannula.

It must also be reiterated that the particular truncated-cone shape of the piston makes possible easier side exit of the product to be dispensed, permits fully emptying the tubular body and prevents any product back flows inside the cannula.

The invention thus conceived is susceptible to numerous modifications and variations.

Furthermore all the details can be replaced with others that are technically equivalent.

In practice, the materials used, as well as the contingent shapes and dimensions, may be any according to requirements without because of this moving outside the protection scope of the following claims.

## Claims

1. Cannula (1) for dispensing fluid products, particularly for rectal applications, comprising:
- at least one tubular body (2) for containing a fluid product (3) to be dispensed which has at least one open axial extremity (6) and a plurality of product dispensing mouths (4) which are provided on the side surface of said tubular body (2) and are split up into at least a first series distributed longitudinally on said tubular body (2), said tubular body (2) comprising:
- a first portion (8), with a substantially cylindrical shape, and
- a second portion (9), with a substantially truncated-cone shape, which extends from said first portion (8),
- at least one cap (17) fittable substantially to measure around said tubular body (2) to temporarily close said dispensing mouths (4),
- at least one piston (7) fitted axially sliding in said tubular body (2) between an initial packaging configuration and a final dispensing configuration, said piston (7) comprising:
- a first section (10), with a substantially cylindrical shape, that slides inside said first portion (8) of the tubular body (2), and
- a second section (11), with a substantially truncated-cone shape, which protrudes from said first section (10) and, in the final dispensing configuration, can be placed substantially in line with the inner surface of said second portion (9) of the tubular body (2),
- at least one push rod (21) for actuation of said piston (7) and fittable in said tubular body (2) through said open axial extremity (6), between said piston (7) and said push rod (21) being interposed temporary mutual fastening means (22, 23), for reversible attachment of the piston to the push rod wherein
- in said initial packaging configuration said first section (10) of the piston (7) is fitted in said first portion (8) of the tubular body (2),
- all said dispensing mouths (4) are provided only along said second portion (9), and
- in said final dispensing configuration the outer surface of the second section (11) of the piston (7) is substantially contiguous with the inner surface of the second portion (9) of the tubular body (2), the shape of said second portion (9) and of said second section (11) allowing obtaining the complete occlusion of all said dispensing mouths (4) once the final complete dispensing configuration has been achieved, so as to prevent any return flows of said product (3) inside said second portion (9).

2. Cannula (1) according to claim 1, **characterized in that** said push rod (21) is removably attachable to said piston (7).

3. Cannula (1) according to claim 1 or 2, **characterized in that** said dispensing mouths (4) are split up into at least a second series distributed longitudinally on said tubular body (2) on a diametrically opposite side with respect to said first series.

4. Cannula (1) according to one or more of the previous claims, **characterized in that** it comprises temporary retaining means (12) for temporarily retaining said piston (7) in said initial packaging configuration which are interposed between said tubular body (2) and said piston (7).

5. Cannula (1) according to claim 4, **characterized in that** said temporary retaining means (12) comprise at least one strip (13) fitted around said open axial extremity (6) and associated with said piston (7) along predetermined fracture areas (14).

6. Cannula (1) according to claim 5, **characterized in that** said strip (13) is of the tear-off type.

7. Cannula (1) according to claim 5, **characterized in that** said strip (13) comprises at least a grip and tear-off flap (15) suitable for breaking said strip (13) to remove it from said tubular body (2).

8. Cannula (1) according to one or more of the previous claims, **characterized in that** it comprises first anti-tampering means (16) for sealing said initial packaging configuration, which are placed in between said tubular body (2) and said piston (7).

9. Cannula (1) according to claim 8, **characterized in that** said first anti-tampering means (16) coincide with said temporary retaining means (12).

10. Cannula (1) according to one or more of the previous claims, **characterized in that** it comprises second anti-tampering means (18) for sealing the closure of said dispensing mouths (4) suitable for providing evidence of the separation of said cap (17) from said tubular body (2),

11. Cannula (1) according to claim 10, **characterized in that** said second anti-tampering means (18) comprise at least an annular element that is associated with the edge of said cap (17) along a tearing line (19) and which can be fixed around said tubular body (2).

12. Cannula (1) according to claim 11, **characterized in that** second anti-tampering means (18) comprise at least a plate (20) associated around said tubular body (2) and with at least a groove in which said annular element (18) can be interlocked.

13. Cannula (1) according to one or more of the previous claims, **characterized in that** said temporary mutual fastening means (22, 23) comprise at least one shaped cavity (22) provided inside at least one of said piston (7) and said push rod (21) and at least one shaped protrusion (23) provided on the other of said piston (7) and said push rod (21) and which can be interlocked into said cavity (22).

14. Cannula (1) according to claim 13, **characterized in that** said cavity (22) is provided in said first section (10) of the piston (7).

15. Cannula (1) according to claim 13 or 14, **characterized in that** said protrusion (23) is provided at an extremity of said push rod (21).

16. Cannula (1) according to one or more of the previous claims, **characterized in that** it comprises grip means (24, 25) for gripping said push rod (21).

17. Cannula (1) according to claim 16, **characterized in that** said grip means (24, 25) comprise at least one pad (25) which extends crossways from said push rod (21).

18. Cannula (1) according to one or more of the previous claims, **characterized in that** said tubular body (2) is at least in part pre-filled with said fluid product (3).

19. Cannula (1) according to one or more of the previous claims, **characterized in that** it contains a unit dosage of fluid product (3) for administration as a single dose.

## Patentansprüche

1. Kanüle (1) zum Abgeben von fluiden Produkten, insbesondere für rektale Anwendungen, umfassend:
- mindestens einen rohrförmigen Körper (2) zur Aufnahme eines abzugebenden fluiden Produkts (3), der mindestens ein offenes, axiales Ende (6) und eine Vielzahl von Produktabgabeöffnungen (4) aufweist, die an der Seitenfläche des rohrförmigen Körpers (2) bereitgestellt und in mindestens eine erste Reihe aufgeteilt sind, die der Länge nach an dem rohrförmigen Körper (2) verteilt sind, wobei der rohrförmige Körper (2) umfasst:
- einen ersten Abschnitt (8) mit einer im Wesentlichen zylindrischen Form, und
- mindestens einen zweiten Abschnitt (9) mit einer im Wesentlichen kegelstumpfartigen Form, die sich von dem ersten Abschnitt (8) erstreckt,
- mindestens eine Kappe (17), die anbringbar ist, um im Wesentlichen den rohrförmigen Körper (2) zum vorübergehenden Verschließen der Abgabeöffnungen (4) rundherum zu umgeben,
- mindestens einen Kolben (7), der eingepasst ist, um in axialer Richtung in dem rohrförmigen Körper (2) zwischen einer anfänglichen Verpackungskonfiguration und einer finalen Abgabekonfiguration zu gleiten, wobei der Kolben (7) umfasst:
- ein erstes Teilstück (10) mit einer im Wesentlichen zylindrischen Form, das innerhalb des ersten Abschnitts (8) des rohrförmigen Körpers (2) gleitet, und
- ein zweites Teilstück (11) mit einer im Wesentlichen kegelstumpfartigen Form, das aus dem ersten Teilstück (10) herausragt und das in der finalen Abgabekonfiguration im Wesentlichen mit der Innenfläche des zweiten Abschnitts (9) des rohrförmigen Körpers (2) ausgerichtet angeordnet werden kann,
- mindestens eine Schubstange (21) zum Betätigen des Kolbens (7) und einsetzbar in den rohrförmigen Körper (2) durch das offene axiale Ende (6), wobei zwischen dem Kolben (7) und der Schubstange (21) vorübergehende gegenseitige Befestigungsmittel (22, 23) zur lösbaren Befestigung von dem Kolben an der Schubstange angeordnet sind, wobei
- in der anfänglichen Verpackungskonfiguration das erste Teilstück (10) des Kolbens (7) in den ersten Abschnitt (8) des rohrförmigen Körpers (2) eingepasst ist,
- alle Abgabeöffnungen (4) nur entlang des zweiten Abschnitts (9) bereitgestellt sind, und
- in der finalen Abgabekonfiguration die Außenfläche des zweiten Teilstücks (11) des Kolbens (7) im Wesentlichen angrenzend an die Innenfläche des zweiten Abschnitts (9) des rohrförmigen Körpers (2) ist, wobei die Form des zweiten Abschnitts (9) und des zweiten Teilstücks (11) das Erhalten des vollständigen Verschlusses von allen Abgabeöffnungen (4) ermöglicht, sobald die endgültige finale Abgabekonfiguration erreicht worden ist, um so jegliches Rückströmen des Produktes (3) in den zweiten Abschnitt (9) zu verhindern.

2. Kanüle (1) gemäß Anspruch 1, **dadurch gekennzeichnet, dass** die Schubstange (21) abnehmbar an dem Kolben (7) befestigbar ist.

3. Kanüle (1) gemäß Anspruch 1 oder 2, **dadurch gekennzeichnet, dass** die Abgabeöffnungen (4) in mindestens eine zweite Reihe aufgeteilt sind, die in Längsrichtung auf dem rohrförmigen Körper (2) auf einer diametral gegenüberliegenden Seite in Bezug auf die erste Reihe verteilt sind.

4. Kanüle (1) gemäß einem oder mehreren der vorhergehenden Ansprüche, **dadurch gekennzeichnet, dass** sie vorübergehende Haltemittel (12), die zwischen dem rohrförmigen Körper (2) und dem Kolben (7) angeordnet sind, zum vorübergehenden Halten des Kolbens (7) in der anfänglichen Verpackungskonfiguration aufweist.

5. Kanüle (1) gemäß Anspruch 4, **dadurch gekennzeichnet, dass** die temporären Haltemittel (12) mindestens einen Streifen (13) aufweisen, der um das offene axiale Ende (6) angebracht und mit dem Kolben (7) entlang von Sollbruchbereichen (14) verbunden ist.

6. Kanüle (1) gemäß Anspruch 5, **dadurch gekennzeichnet, dass** der Streifen (13) vom Abreißtyp ist.

7. Kanüle (1) gemäß Anspruch 5, **dadurch gekennzeichnet, dass** der Streifen (13) mindestens eine Griff- und Abreißlasche (15) aufweist, die zum Zerbrechen des Streifens (13) geeignet ist, um ihn von dem rohrförmigen Körper (2) zu entfernen.

8. Kanüle (1) gemäß einem oder mehreren der vorhergehenden Ansprüche, **dadurch gekennzeichnet, dass** sie erste Anti-Manipulationsmittel (16) zur Versiegelung der anfänglichen Verpackungskonfiguration aufweist, die zwischen dem rohrförmigen Körper (2) und dem Kolben (7) angeordnet sind.

9. Kanüle (1) gemäß Anspruch 8, **dadurch gekennzeichnet, dass** die ersten Anti-Manipulationsmittel (16) mit den temporären Haltemitteln (12) übereinstimmen.

10. Kanüle (1) gemäß einem oder mehreren der vorhergehenden Ansprüche, **dadurch gekennzeichnet, dass** sie zweite Anti-Manipulationsmittel (18) zum Versiegeln des Verschlusses der Abgabeöffnungen (4) aufweist, die für das Erbringen des Nachweises der Abtrennung der Kappe (17) von dem rohrförmigen Körper (2) geeignet sind.

11. Kanüle (1) gemäß Anspruch 10, **dadurch gekennzeichnet, dass** die zweiten Anti-Manipulationsmittel (18) mindestens ein ringförmiges Element aufweisen, das mit dem Rand der Kappe (17) entlang einer Abreißlinie (19) verbunden ist und das um den rohrförmigen Körper (2) befestigt werden kann.

12. Kanüle (1) gemäß Anspruch 11, **dadurch gekennzeichnet, dass** die zweiten Anti-Manipulationsmittel (18) mindestens eine Platte (20) aufweisen, die rund um den rohrförmigen Körper (2) angebracht ist und mindestens eine Nut aufweist, in welcher das ringförmige Element (18) verriegelt werden kann.

13. Kanüle (1) gemäß einem oder mehreren der vorhergehenden Ansprüche, **dadurch gekennzeichnet, dass** die temporären gegenseitigen Befestigungsmittel (22, 23) mindestens einen geformten Hohlraum (22), der im Inneren von mindestens einem von dem Kolben (7) und der Schubstange (21) vorgesehen ist, und mindestens einen geformten Vorsprung(23) aufweisen, der auf dem anderen von dem Kolben (7) und der Schubstange (21) vorgesehen ist, und der in dem Hohlraum (22) verriegelt werden kann.

14. Kanüle (1) gemäß Anspruch 13, **dadurch gekennzeichnet, dass** der Hohlraum (22) in dem ersten Teilstück (10) des Kolbens (7) vorgesehen ist.

15. Kanüle (1) gemäß Anspruch 13 oder 14, **dadurch gekennzeichnet, dass** der Vorsprung (23) an einem Ende der Schubstange (21) vorgesehen ist.

16. Kanüle (1) gemäß einem oder mehreren der vorhergehenden Ansprüche, **dadurch gekennzeichnet, dass** sie Greifmittel (24, 25) zum Greifen der Schubstange (21) aufweist.

17. Kanüle (1) gemäß Anspruch 16, **dadurch gekennzeichnet, dass** die Greifmittel (24, 25) mindestens eine Scheibe (25) aufweisen, die sich quer zu der Schubstange (21) erstreckt.

18. Kanüle (1) gemäß einem oder mehreren der vorhergehenden Ansprüche, **dadurch gekennzeichnet, dass** der rohrförmige Körper (2) mindestens teilweise mit dem fluiden Produkt (3) vorgefüllt ist.

19. Kanüle (1) gemäß einem oder mehreren der vorhergehenden Ansprüche, **dadurch gekennzeichnet, dass** sie eine Einheitsdosis des fluiden Produkts (3) zur Verabreichung als eine Einzeldosis enthält.

## Revendications

1. Canule (1) pour l'administration de produits fluides, en particulier pour des applications rectales, comprenant :
- au moins un corps tubulaire (2) destiné à contenir un produit fluide (3) à administrer possédant au moins une extrémité axiale ouverte (6) et une pluralité de bouches de distribution de produit (4) qui sont prévues sur la surface latérale dudit corps tubulaire (2) et sont réparties en au moins une première série répartie longitudinalement sur ledit corps tubulaire (2), ledit corps tubulaire (2) comprenant :
- une première portion (8), de forme sensiblement cylindrique, et
- une seconde portion (9), de forme sensiblement tronconique, qui s'étend à partir de ladite première portion (8),
- au moins un capuchon (17) pouvant s'adapter sensiblement sur mesure autour dudit corps tubulaire (2) pour obturer temporairement lesdites bouches de distribution (4),
- au moins un piston (7) monté axialement coulissant dans ledit corps tubulaire (2) entre une configuration initiale de conditionnement et une configuration finale de distribution, ledit piston (7) comprenant :
- une première section (10), de forme sensiblement cylindrique, qui coulisse à l'intérieur de ladite première portion (8) du corps tubulaire (2), et
- une seconde section (11), de forme sensiblement tronconique, qui fait saillie depuis ladite première section (10) et, dans la configuration finale de distribution, peut être placée sensiblement en alignement avec la surface intérieure de ladite seconde portion (9) du corps tubulaire (2),
- au moins une tige poussoir (21) pour l'actionnement dudit piston (7) et pouvant s'adapter dans ledit corps tubulaire (2) à travers ladite extrémité axiale ouverte (6), entre ledit piston (7) et ladite tige poussoir (21) étant interposés des moyens de solidarisation mutuelle temporaires (22, 23), pour la fixation réversible du piston sur la tige poussoir dans laquelle :
- dans ladite configuration initiale de conditionnement ladite première section (10) du piston (7) est montée dans ladite première portion (8) du corps tubulaire (2),
- toutes lesdites bouches de distribution (4) sont prévues uniquement le long de ladite seconde portion (9), et
- dans ladite configuration finale de distribution la surface extérieure de la seconde section (11) du piston (7) est sensiblement jointive avec la surface intérieure de la seconde portion (9) du corps tubulaire (2), la forme de ladite seconde portion (9) et de ladite seconde section (11) permettant d'obtenir l'occlusion complète de toutes lesdites bouches de distribution (4) une fois que la configuration finale de distribution complète a été réalisée, afin d'empêcher tout retour d'écoulement dudit produit (3) à l'intérieur de ladite seconde portion (9).

2. Canule (1) selon la revendication 1, **caractérisée en ce que** ladite tige poussoir (21) est apte à être fixée de façon amovible sur ledit piston (7).

3. Canule (1) selon la revendication 1 ou 2, **caractérisée en ce que** lesdites bouches de distribution (4) sont réparties en au moins une seconde série répartie longitudinalement sur ledit corps tubulaire (2) sur une face diamétralement opposée par rapport à ladite première série.

4. Canule (1) selon l'une ou plusieurs des revendications précédentes, **caractérisée en ce qu'**elle comprend des moyens de retenue temporaire (12) pour temporairement retenir ledit piston (7) dans ladite configuration initiale de conditionnement qui sont interposés entre ledit corps tubulaire (2) et ledit piston (7).

5. Canule (1) selon la revendication 4, **caractérisée en ce que** lesdits moyens de retenue temporaire (12) comprennent au moins une bande (13) montée autour de ladite extrémité axiale ouverte (6) et associée audit piston (7) le long de zones de rupture prédéterminées (14).

6. Canule (1) selon la revendication 5, **caractérisée en ce que** ladite bande (13) est du type à arracher.

7. Canule (1) selon la revendication 5, **caractérisée en ce que** ladite bande (13) comprend au moins une languette à saisir et à arracher (15) apte à rompre ladite bande (13) pour l'enlever dudit corps tubulaire (2).

8. Canule (1) selon l'une ou plusieurs des revendications précédentes, **caractérisée en ce qu'**elle comprend des premiers moyens d'inviolabilité (16) pour sceller ladite configuration initiale de conditionnement, qui sont placés entre ledit corps tubulaire (2) et ledit piston (7).

9. Canule (1) selon la revendication 8, **caractérisée en ce que** lesdits premiers moyens d'inviolabilité (16) coïncident avec lesdits moyens de retenue temporaire (12).

10. Canule (1) selon l'une ou plusieurs des revendications précédentes, **caractérisée en ce qu'**elle comprend des seconds moyens d'inviolabilité (18) pour sceller l'obturation desdites bouches de distribution (4) aptes à mettre en évidence la séparation dudit capuchon (17) d'avec ledit corps tubulaire (2).

11. Canule (1) selon la revendication 10, **caractérisée en ce que** lesdits seconds moyens d'inviolabilité (18) comprennent au moins un élément annulaire qui est associé au bord dudit capuchon (17) le long d'une ligne d'arrachage (19) et qui peut être fixé autour dudit corps tubulaire (2).

12. Canule (1) selon la revendication 11, **caractérisée en ce que** les seconds moyens d'inviolabilité (18) comprennent au moins une plaque (20) associée autour dudit corps tubulaire (2) et avec au moins une rainure dans laquelle ledit élément annulaire (18) peut être verrouillé,

13. Canule (1) selon l'une ou plusieurs des revendications précédentes, **caractérisée en ce que** lesdits moyens de solidarisation mutuelle temporaires (22, 23) comprennent au moins une cavité conformée (22) prévue à l'intérieur d'au moins l'un parmi ledit piston (7) et ladite tige poussoir (21) et au moins une protubérance conformée (23) prévue sur l'autre parmi ledit piston (7) et ladite tige poussoir (21) et qui peut être verrouillée dans ladite cavité (22).

14. Canule (1) selon la revendication 13, **caractérisée en ce que** ladite cavité (22) est prévue dans ladite première section (10) du piston (7).

15. Canule (1) selon la revendication 13 ou 14, **caractérisée en ce que** ladite protubérance (23) est prévue à une extrémité de ladite tige poussoir (21).

16. Canule (1) selon l'une ou plusieurs des revendications précédentes, **caractérisée en ce qu'**elle comprend des moyens de préhension (24, 25) pour la préhension de ladite tige poussoir (21).

17. Canule (1) selon la revendication 16, **caractérisée en ce que** lesdits moyens de préhension (24, 25) comprennent au moins un patin (25) qui s'étend transversalement à partir de ladite tige poussoir (21).

18. Canule (1) selon l'une ou plusieurs des revendications précédentes, **caractérisée en ce que** ledit corps tubulaire (2) est au moins partiellement pré-rempli dudit produit fluide (3).

19. Canule (1) selon l'une ou plusieurs des revendications précédentes, **caractérisée en ce qu'**elle contient un dosage unitaire de produit fluide (3) pour une administration en une seule prise.
